Europäisches Patentamt

European Patent Office

Office européen des brevets

(11) Publication number: **0 337 243**
**A1**

# EUROPEAN PATENT APPLICATION

(21) Application number: **89105852.1**

(22) Date of filing: **04.04.89**

(51) Int. Cl.⁴: **C12P 21/02 , C07K 3/08 , C07K 3/12**

(30) Priority: **14.04.88 US 181337**

(43) Date of publication of application:
**18.10.89 Bulletin 89/42**

(84) Designated Contracting States:
**AT BE CH DE FR GB IT LI NL**

(71) Applicant: **F. HOFFMANN-LA ROCHE AG**
**Postfach 3255**
**CH-4002 Basel(CH)**

(72) Inventor: **Khan, Fazal R.**
**205 Vanhouten Avenue**
**Passaic N.J. 07055(US)**
Inventor: **Umagat, Herminia J.**
**500 Rifle Camp Road**
**West Paterson N.J. 07424(US)**

(74) Representative: **Mezger, Wolfgang, Dr. et al**
**Grenzacherstrasse 124 Postfach 3255**
**CH-4002 Basel(CH)**

(54) Process for purifying recombinant human interleukin-2.

(57) Methods are provided for the purification of recombinant human IL-2 using a tandem two-column reversed phase liquid chromatography system. By the use of this system, homogeneous IL-2 can be produced, as judged by analytical reversed phase HPLC and by SDS polyacrylamide gel electrophoretic analysis. An optional method for the removal of acetonitrile and endotoxin from the purified IL-2 is also provided.

EP 0 337 243 A1

## Process for purifying recombinant human interleukin-2

This invention relates to recombinant human interleukin-2 and to methods for purifying this protein by reversed phase liquid chromatography.

Interleukin-2 (IL-2) is a soluble protein which is capable of modulating lymphocyte reactivity and promoting the long-term in vitro culture of antigen-specific effector T-lymphocytes. In the past, IL-2 has been produced primarily from mammalian cells that are capable of synthesizing the protein, after such cells have been stimulated with a mitogen. For example. Morgan et al. [Science 193:1007 (1976)] and Ruscetti et al. [J. Immunol. 119:131 (1977)] have recovered IL-2 from pooled normal human lymphocytes that had been stimulated with phytohemagglutinin, while Gillis et al. [Nature 268:154 (1977)] used normal DBA/2 mouse spleen cells stimulated with concanavalin A as a source of the protein. More recently, Stern [United States Patent No. 4,490,289] has described the use of induced human malignant cells as a source of IL-2.

Efforts have also been made to produce IL-2 through the use of recombinant DNA methodology. For example, Taniguchi et al. [Nature 302:305 (1983)] have described the cloning and expression of a complementary DNA (cDNA) coding for human IL-2 prepared from messenger RNA from the Jurkat leukemia cell line. The expression of IL-2 was carried out by Taniguchi et al. in cultured monkey COS cells, although the authors stated that work on the expression of IL-2 rDNA in E. coli was in progress, and that from the E. coli system it would soon be possible to produce IL-2 in large quantities. Taniguchi et al. have also disclosed the nucleotide sequence of the CDNA coding for human IL-2.

Rosenberg et al. [Science 223:1412 (1984)] have expressed IL-2 in E. coli, using a gene isolated from the Jurkat cell line. More recently, Souza et al. [European Patent Application, Publication No. 136 489] have described the cloning and expression in microorganisms of chemically synthesized DNA sequences comprising structural genes coding for a polypeptide having the amino acid sequence and properties of IL-2. Souza et al. also disclose the use of synthetic genes to produce IL-2 polypeptide analogs which differ in amino acid sequence from the natural IL-2 polypeptide. In the examples provided in the Souza et al. patent application, E. coli is the host organism.

When recombinant human IL-2 is expressed in E. coli, it is generally produced in form of insoluble aggregates. The IL-2 must be extracted from these aggregates (called inclusion bodies) and then purified in some way. Weir et al. [J.

Chromatogr. 396:209 (1987)] have used a combination of gel filtration chromatography, ion exchange chromatography and reversed phase high-performance liquid chromatography (HPLC) to purify such IL-2.

Koths et al. [U.S. Patent No. 4,569,790] have purified recombinant IL-2 from bacteria by extracting the insoluble fraction from a lysate of the bacteria with a solubilizing agent in the presence of a reducing agent, and then purifying the solubilized IL-2 after a reoxidation step by gel filtration or reversed phase HPLC or a combination of the two. The solubilizing agents used were preferably sodium dodecyl sulfate (SDS) or sodium lauryl sarcosine.

The present invention provides methods by which extracts of bacterial cells containing recombinant human IL-2 are purified to homogeneity characterized in that a tandem two-column reversed phase liquid chromatography system is used.

In a preferred embodiment of the invention recombinant human IL-2 is purified by a process comprising:

(a) disrupting cultured bacterial cells containing a vector capable of directing the expression of recombinant human IL-2, to produce a bacterial cell lysate containing IL-2;

(b) isolating an insoluble fraction from the bacterial cell lysate;

(c) extracting the isolated insoluble fraction from the bacterial cell lysate with a solution of from about 4 to 7 M guanidine-HCl to obtain an IL-2 containing extract;

(d) diluting the IL-2 containing extract from about 10-to about 1,000-fold with a guanidine-HCl free buffer;

(e) applying the diluted IL-2 containing extract to a first reversed phase column, whereby the IL-2 is retained by the first column;

(f) eluting the IL-2 from the first column with an increasing acetonitrile gradient and pooling fractions containing IL-2;

(g) applying the pooled IL-2 containing fractions from the first column to a second reversed phase column, whereby the IL-2 is retained by the second column; and

(h) eluting the IL-2 from the second column with an increasing acetonitrile gradient and pooling fractions containing IL-2.

The guanidine-HCl free buffer may be any buffer which is suitable for use in biological systems. Many such buffers are known to the man skilled in the art. Examples for such buffers are buffers containing tris-(hydroxymethyl)aminomethane (Tris). Most preferably the IL-2 containing extract is diluted about 40-fold with a guanidine-HCl free buffer such as 0.01M Tris-HCl (pH 7.9). 0.035M NaCl.

In another preferred embodiment of the invention, extraction step (c) above is preceded by a step comprising extracting the isolated insoluble fraction of step (b) with a solution containing about 1.75 M guanidine-HCl and discarding the extract.

In still another preferred embodiment of the invention, the diluted IL-2 containing extract of step (d) above is allowed to stand for a period of time ranging from about four hours to about two days at about 2 to 8°C prior to application to the first reversed phase column. Preferably, the extract is allowed to stand for about 8 to 24 hours.

In still another embodiment, the pooled IL-2 from the second reversed phase column above is further purified to remove acetonitrile and endotoxin by chromatography in a carboxymethyl ion exchange column.

The present invention can be more readily understood by reference to the detailed description below and to the following figures, in which:

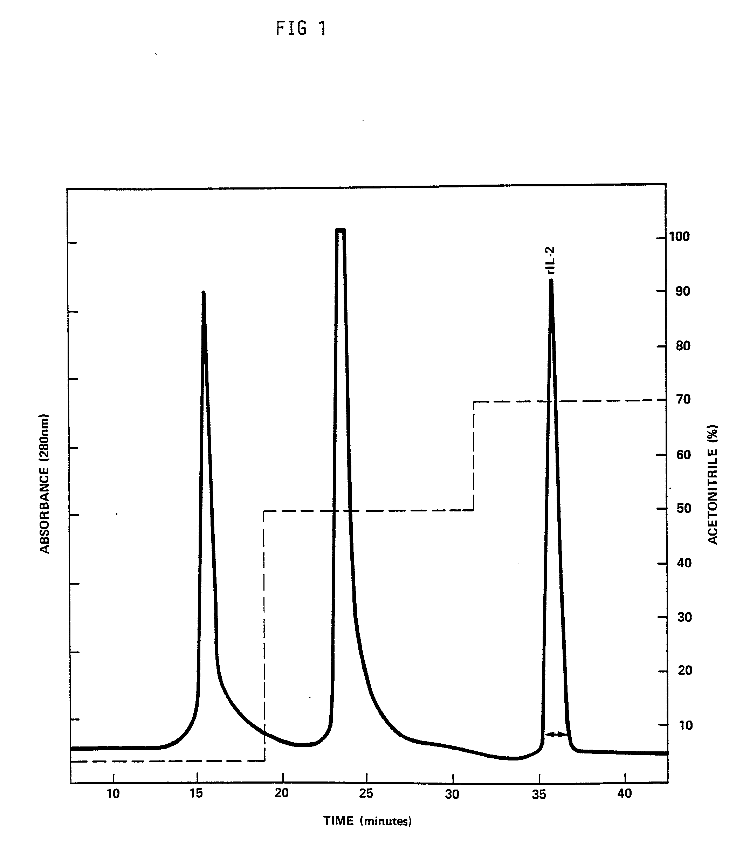

Fig. 1 is an elution profile for the purification of recombinant human IL-2 from a first reversed phase liquid chromatography column, showing the absorbance at 280 nm as a function of time and the buffer/acetonitrile step gradient used to elute the protein. The peak containing the IL-2 is indicated by the double arrow.

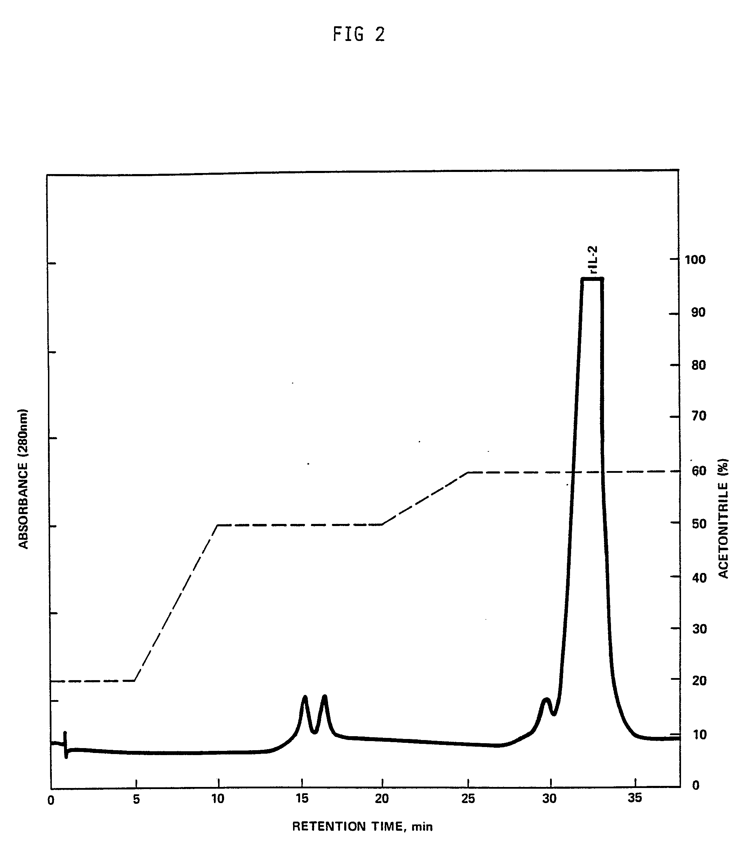

Fig. 2 is an elution profile for the purification of recombinant human IL-2 from a second reversed phase column, showing the absorbance at 280 nm as a function of time and the buffer/acetonitrile gradient used to elute the protein. The IL-2 was applied to the column in form of the pooled IL-2 containing fractions obtained from the purification step shown in Fig. 1.

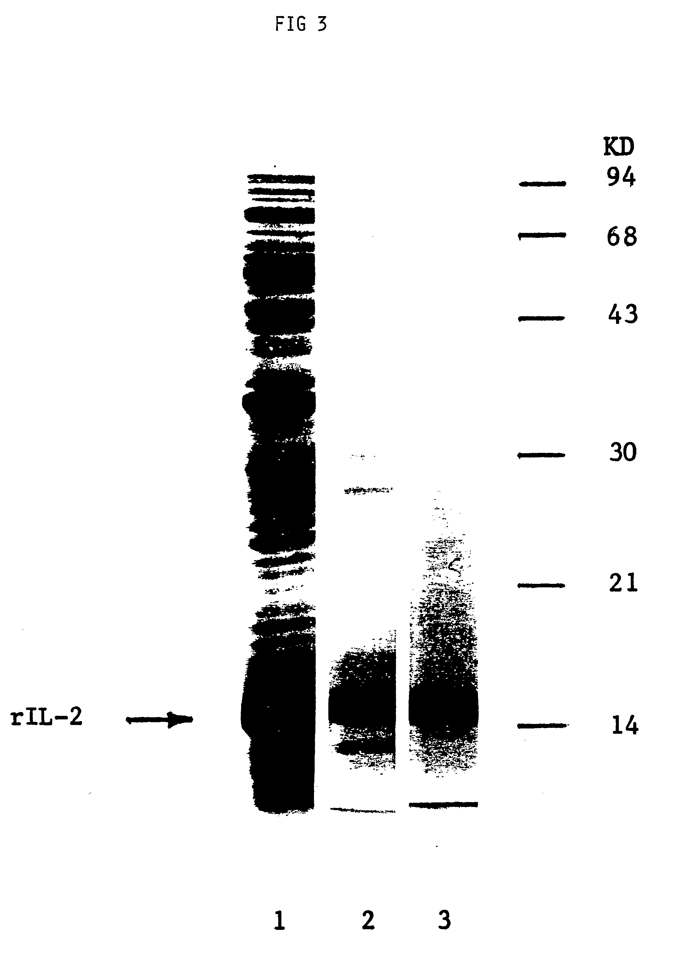

Fig. 3 shows an SDS polyacrylamide gel electrophoretic analysis of a sample from the crude extract from E. coli cells producing human IL-2 (lane 1), a sample from the pooled IL-2 containing fractions recovered from the purification step shown in Fig. 1 (lane 2) and a sample from the pooled IL-2 containing fractions recovered from the purification step shown in Fig. 2. The position of the IL-2 band and the positions of standard molecular weight marker proteins run in the same gel system are shown. The molecular weight of the marker proteins are indicated in kilo daltons (KD) whereby 1 KD = 1'000 dalton.

The present invention achieves superior purification of recombinant human IL-2 by the use of a tandem two-column reversed phase liquid chromatography system. The IL-2 obtained by using this system is homogeneous by SDS polyacrylamide gel electrophoretic analysis and by analytical reversed phase HPLC. The chromatography system of the present invention is advantageous in that gram quantities of protein can be purified, and in that the system can be readily automated for repetitive chromatography and column recycling.

As used herein, the term "recombinant human IL-2" means an unglycosylated protein produced by a microorganism containing a human IL-2 gene or a modification of such a gene encoding a protein having an amino acid sequence that is at least substantially similar to the amino acid sequence of native human IL-2 and has biological activity similar to that of native human IL 2. Substantial similarity of amino acid sequence means that the amino acid sequence encoded by the modified gene differs by deletion, addition and/or substitution of one or more amino acids from the amino acid sequence of native human IL-2 without having a substantial adverse effect on the bioactivity of the IL-2 protein.

The microorganisms, e.g. bacteria containing human IL-2 are microorganisms containing a recombinant vector or plasmid in which a human IL-2 gene is operatively linked to an expression control sequence. Such microorganisms are capable of expressing the human IL-2 gene. They are obtained by methods well known in the art, e.g. by transformation with such recombinant vectors or plasmids. The microorganisms can be grown in a suitable culture media using methods well known in the art. After sufficient quantities of the cells have accumulated, the cells can be separated from the culture medium e.g., by centrifugation. A cell paste containing the microorganisms in concentrated form obtained in this way is suitable for cell disruption.

The microorganisms producing the recombinant IL-2 can be disrupted using methods commonly employed in the art such as sonication, French pressure cell or Gaulin homogenizer disruption (APV Gaulin, Everett, Massachusetts, U.S.A.) or other mechanical means (Charm et al., Meth. Enzymol. 22 476-556 [1971]), osmotic shock or repeated freezing and thawing. The major purpose of such disruption is to facilitate the subsequent extraction and solubilization steps. If desired, a protease inhibitor such as phenylmethylsulfonyl fluoride can be added to the cell paste prior to disruption.

Following disruption, the insoluble fraction of the cell lysate is isolated from the soluble constituents. This isolation can be conveniently accomplished by centrifuging the lysate at from about 5,000 to about 12,000 x g for about 1 hour. Preferably, the temperature is maintained at about 2-8°C during the isolation of the insoluble fraction and during the later purification steps.

Extraction of the insoluble fraction of the cell lysate may be carried out in two steps. In the first step, bacterial proteins other than the recombinant IL-2 are extracted, leaving most of the IL-2 behind in the insoluble material. This extraction is carried out using a solution of from about 1.75 to 2 M guanidine-HCl. Mechanical agitation such as shaking or stirring is preferably applied during the extraction step. A prior extraction step can also be carried out using a buffer of about pH 8, since such a step also removes contaminating bacterial proteins. In any agent the soluble extracts from such steps are discarded.

The remaining insoluble material containing most of the IL-2 is then extracted with a solution of from about 4 to 7 M guanidine-HCl preferably 7 M guanidine-HCl. The extract obtained is this way is diluted about 10- to about 1,000-fold with a guanidine-HCl free buffer. A buffer containing 10 mM Tris-HCl, 0.035 M NaCl pH 7.9, was used in the example below, but any similar buffer would be acceptable.

Following dilution of the extracted flocculent material which contains primarily contaminating proteins develops upon standing. The amount of this material increases with time up to about 2 days after dilution. The flocculent material is removed by centrifugation or filtration and is discarded. In this way contaminating proteins are removed with little or no loss of IL-2. The diluted extract is now preferably allowed to stand at 2-8° C for from 4 hours to about 2 days prior to further purification.

Reversed phase chromatography is carried out using resins containing alkyl chains bonded to a suitable support matrix. Suitable column materials are articles of commerce. Preferred column materials are the C4 and C18 silica support materials of VYDAC, Hesperia, California U.S.A. These materials contain butyl or octadecyl silane groups covalently bonded by means of a siloxane (silicon-oxygen-silicon) bond to the surface of 250-300 Angstrom pore diameter silica particles which have been classified to mean particle sizes of from 10 to 30 μm.

Purification of the diluted IL-2 containing extract is performed using two reversed phase chromatography columns. Preferably, the first column contains larger 20-30 μm particle size support material to produce lower back pressure and higher flow rates. The second column preferably contains finer support material having a 10-20 μm particle size, for higher resolution.

Prior to applying the diluted IL-2 containing extract to the first column ,the extract is preferably acifified using an appropriate acid such as acetic acid. A pH of the extract of about 3.5 is preferred. The diluted extract can also be concentrated about ten-fold by ultrafiltration prior to application to the first reversed phase column, although such concentration is not essential.

The elution of proteins from the columns is carried out in a manner well known in the art. For example, a linear, concave or convex gradient of increasing acetonitrile could be used. Preferably, a step gradient is used as explained more fully below. The advantage of such a step gradient is that it is readily adapted to automatic programmed operation. Through such operation, relatively large quantities of IL-2 can be produced through cyclic column equilibration and regeneration, sample application and elution.

Elution of proteins from the columns can be conveniently monitored using known detection methods. For example, an automated fluorescence detection system such as the one described by Stein et al. [Methods in Enzymology 78:435 (1981)] can be employed. Preferably, a non-destructive method such as monitoring ultraviolet absorbance at 28o nanometers is used. A suitable automated UV absorbance detector is available from Waters Associates, Milford, Massachusetts, U.S.A.

The presence of IL-2 in the eluate can be monitored using standard IL-2 bioassays of fraction aliquots or, preferably, by analytical HPLC using known IL-2 as a standard.

By carrying out the reversed phase chromatography process in two steps in accordance with the presence invention, recombinant human IL-2 has been purified from microorganisms to homogeneity as shown by an analytical reversed phase HPLC and by SDS polyacrylamide gel electrophoresis. Such homogeneity of the IL-2 has been routinely achieved even though there are peaks of contaminating proteins that elute close to the IL-2 from the reversed phase columns.

Koths et al. [U.S, patent No. 4,569,790] have disclosed that the reversed phase columns used in this invention can also effectively remove bacterial endotoxin contamination. Nevertheless, some endotoxin contamination may be present in the purified IL-2. Moreover, acetonitrile will be present in the IL-2 pool. Both of these contaminants can be removed using methods known in the art.

For example, they can be removed by gel filtration chromatography in Sephadex® G-50 (Pharmacia, Uppsala, Sweden). Alternatively, and preferably, any endotoxin and the acetonitrile remaining in the purified IL-2. after reversed phase chromatography is removed by ion exchange chromatography in a carboxymethyl resin. Carboxymethyl cellulose or other carboxymethyl-substituted resins can be used for this purpose, although CM-Fractogel® [Toyosoda, Tokyo, Japan] is preferred. The IL-2 is eluted from such columns by standard methods.

If desired, mannitol can be added to the purified IL-2 to stabilize the protein against loss of biological activity. Mannitol at a concentration of from about 1 to about 50 mg/ml can be used for this purpose. A concentration of about 5 mg/ml is preferred.

The overall yield of recombinant human IL-2 produced by the methods of this invention generally ranges from about 45 to about 60 percent, in terms of total extract IL-2 protein recovered. The specific bioactivity of the purified IL-2 is typically about $1.6 \times 10^1$ activity units per milligram of protein.


EXAMPLE


The processes of the present invention are further illustrated by the following example.


General Methods


IL-2 bioassays were carried out using a colorimetric modification of the method described by Gillis et al. in J. Immunol. 120:2027 (1978). In this assay CTLL cells were used as target cells. CTLL cells are cells of a murine cytotoxic lymphoid cell line dependent upon IL-2 for growth [Gillis et al., J. Exp. Med. 146:468 (1977)]. The CTLL cells are available from the American Type Culture Collection, 12301 Parklawn Drive, Rockville, Maryland, U.S.A. under the accession number ATCC TIB 214 (CTLL-2) or from Immunex Corp., Seattle, Washington, U.S.A. The cells were maintained in RPMI-1640 medium (Gibco Laboratories, Grand Island, New York, U.S.A.) supplemented with 5% (vol/vol) fetal calf serum, 25 mM HEPES buffered 50 μg/ml gentamicin (Schering/Plough Inc., Bloomfield, New Jersey, U.S.A.) and 200 units/ml crude human IL-2.

For the IL-2 bioassay, CTLL cells were removed from the IL-2 containing maintenance medium by sedimentation for 10 minutes at 500 x g in a Beckman Model J-6B centrifuge (Beckman Instruments, Inc., Palo Alto, California, U.S.A.), and washed twice in HB101 medium (Hana Biologicals, Berkeley, California, U.S.A.) without IL-2. The cells were resuspended in HB101 medium supplemented with 50 μg/ml gentamicin and adjusted to a concentration of $2 \times 10^5$ cells/ml. Samples to be titrated were diluted in HB101 in the first wells of a row in a half-area microtiter plate (Costar No. 3696) to a final volume of 0.1 ml. Subsequent wells in the rows contained 0.05 ml of HB101 medium. Twofold dilutions of the samples were made by transferring 0.05 ml aliquots serially to the end of the rows. Several wells filled with 0.05 ml of medium without IL-2 served as a negative controls. All wells were seeded with 0.05 ml of the cell suspension. Thereupon the plate was covered with a plastic lid and incubated for 20 hours at 37° C in a humidified chamber containing 5% $CO_2$ in air.

The bioassay is based upon a colorimetric determination of lactic acid (LA) produced as an end product of glucose metabolism by stimulated cells. Reagents for this colorimetric determination of LA were obtained from the Sigma Chemical Co., St. Louis, Missouri, U.S.A. Solution A contained 1.6 mg/ml β-nicotinamide adenine dinucleotide and 0.03% (wt/vol) gelatin in a 0.6 M glycine, 80 mM hydrazine buffer at pH 9.2. Solution B contained 166 units/ml lactate dehydrogenase, 0.5 mg/ml p-iodonitrotetrazolium violet, and 0.03 mg/ml phenazine methosulfate in water. A standard solution of LA diluted in phosphate buffered saline served as a positive control for the assay. A solution of 0.35 N HCl was used to stop the reaction. A 96-well microtiter plate which contained 0.05 ml of solution A in each well was used as the reaction plate to determine the amount of LA in a 0.015 ml sample obtained from corresponding wells of the biological assay microtiter plates.

The addition of 0.05 ml of solution B to each well in the reaction plate started the reaction. After sufficient time for the reduction of the tetrazolium dye has elapsed, the reaction was stopped by the addition of 0.05 ml of the HCl solution to each well. The optical density at 550 nm in the reaction solution in each well was then determined with a microplate spectrophotometer.

A unit of IL-2 activity is defined as a quantity of IL-2 which produces a half maximal response in the assay. The IL-2 concentration is calculated as the reciprocal of the dilution in the 50% endpoint well. National Institutes of Health Bureau of Biological Response Modifiers (BRMP) Reference Reagent Human IL-2 (Jurkat) material was used as a standard.

Column fractions were analyzed by analytical HPLC using a Spectra Physics Model SP 8700 solvent delivery system and a Waters Model 440 absorbance detector (Waters Associates, Milford Massachusetts, U.S.A.) set at 280 nm. The column contained C4 support material and had bed dimensions of 4.6 mm diameter x 15 cm length. The column was equilibrated with a 95:5 (vol/vol) mixture of buffer (0.01 M phosphoric acid 0.4 M guanidine-HCl 0.05 M lithium chloride, pH 2.5) and acetonitrile.

After sample application, the proteins were eluted stepwise at a flow rate of 1 ml/minute with the following gradient of increasing acetonitrile:

| Mobile Phase Composition (Buffer: Acetonitrile) | Time (Minutes) |
|---|---|
| 95:5 | 0-3 |
| 50:50 | 6-12 |
| 35:65 | 15-25 |

During the intervals of 3-6 and 12-15 minutes, the concentration of acetonitrile was increased by linear gradients to the indicated levels.

The concentration of IL-2 in the samples was determined by comparing the integrated areas of the IL-2 peaks with the integrated area of a peak produced by a known IL-2 standard sample, chromatographed under the same conditions.

Sodium dodecyl sulfate (SDS) polyacrylamide gel electrophoretic analysis was performed using the method of Laemmli [Nature 227:680 (1970)]. The gels were stained either with Coomassie brilliant blue dye or with silver stain [Switzer et al., Anal. Biochem. 98: 231 (1979)].

## Extraction of IL-2

Recombinant human IL-2 was produced in E. coli cells transformed with the expression plasmid pRC 233/IL 2/Δtet. This plasmid and the methods for its construction are described in detail in Ju et al., J. Biol. Chem. 262:5723 (1987), which is hereby incorporated by reference. E.coli cells transformed with any other known expression plasmid capable of expressing human IL-2 could be used. Transformants containing said plasmid were cultured under standard conditions.

A paste of the transformed E. coli cells was uniformly suspended in 30 mM Tris HCl (pH 8.0) containing 5 mM ethylenediaminetetraacetic acid (EDTA) [Buffer A, 3 ml/g of cell paste] and stirred at 2-8° C. The suspension was passed through a Gaulin homogenizer two or three times at about 7,000 psi (approx. 4,8 x $10^7$ Pascal), with the homogenizer exit temperature maintained at 2-8° C with a cooling coil.

The cell lysate was centrifuged at 12,000 x g for 15-30 minutes in a Sorvall RC-5B centrifuge with a GSA rotor (DuPont, Wilmington, Delaware, U.S.A.). and the supernatant fluid containing soluble proteins was discarded. The pellet was suspended in Buffer A (1 ml/g of cell paste) and stirred for 30 to 60 minutes at about 4° C. After centrifugation as above, the supernatant fluid was discarded.

The pellet was suspended in 1.75 M guanidine-HCl (1-2 ml/g cell paste) and mixed for 30-40 minutes at 2-8° C as described above. The supernatant fluid was discarded, and the pellet was suspended in 7 M guanidine-HCl (2.5 ml/g cell paste) and stirred for 60-90 minutes at 2-8° C. The extract was centrifuged as above, and the resulting supernatant fluid was diluted 40-fold with extract dilution buffer [0.01 M Tris-HCl (pH 7.9), 0.035 M NaCl and allowed to stand for 24 hours at about 4° C. While the solution was standing flocculent debris developed. The diluted supernatant fluid was carefully decanted from the settled flocculent debris, filtered successively through 2.5 and 1.2 μm filters and then concentrated 10-fold in a Millipore Pellicon system (Millipore, Bedford, Massachusetts, U.S.A.).

## Preparative Reversed phase Liquid Chromatography

An 18 cm (inside diameter) column packed to a bed height of 10 cm with VYDAC C4 or C18 resin (250-300 Angstrom pore size, 20-30 μm particle size) was equilibrated with a 95:5 (vol/vol) mixture of buffer (0.01 M phosphoric acid 0.4 M guanidine-HCl 0.05 M lithium chloride, pH 2.5) and acetonitrile. The pH of the filtered IL-2 extract was adjusted to 3.5 with acetic acid. Thereupon a volume of the extract containing 1 g of protein was pumped onto the column material.

Protein was eluted from the column at a flow rate of 400 ml/minute using the following step gradient of increasing acetonitrile:

| Mobile Phase Composition (Buffer: Acetonitrile) | Time (Minutes) |
|---|---|
| 95:5 | 0-18 |
| 50:50 | 18-32 |
| 30:70 | 32-45 |

The column effluent was monitored at a wave-length of 280 nm. 3-liter fractions were collected and then analyzed by HPLC as described above. Fractions containing IL-2 (see elution profile in Fig. 1) were pooled and then used for further purification of the IL-2. Total recovery of IL-2 protein from the first column was 75%.

The IL-2 pool from the above preparative HPLC column was separated into its organic and aqueous phases by cooling to -70°C. A volume of the aqueous phase containing about 400 mg of protein was applied to a 5 cm (inside diameter) HPLC column packed to a bed height of 25 cm with VYDAC C18 resin (300 Angstrom pore size 10-20 μm particle size). This column had previously been equilibrated with an 80:20 (vol/vol) mixture of buffer (0.01 M phosphoric acid. 0.05 M lithium chloride and 0.4 M guanidine-HCl, pH 2.5) and acetonitrile.

protein was eluted from the column at a flow rate of 80 ml/minute using the following step gradient of increasing acetonitrile:

| Mobile Phase Composition (Buffer: Acetonitrile) | Time (Minutes) |
|---|---|
| 80:20 | 0-5 |
| 50:50 | 10-20 |
| 40:60 | 25-40 |

During the intervals of 5-10 and 20-25 minutes, the concentration of acetonitrile was increased by linear gradients to the indicated levels.

The column effluent was monitored at a wave length of 280 nm, and about 450-ml fractions were collected and analyzed by HPLC as described above. Fractions containing IL-2 were pooled (see Fig. 2).

Aliquots of the IL-2 pools from this and the prior preparative reversed phase column were subjected to SDS polyacrylamide gel electrophoretic analysis, with the results shown in Fig. 3. Lanes 1-3 contained a sample from the crude E. coli extract prior to application to the first reversed phase column, a sample from the IL-2 pool from the first column and a sample from the IL-2 pool from the second column, respectively. About 20 μg of IL-2 were present in each lane.

It can be seen from Fig. 3 that the IL-2 from the second column migrated as a single protein band in the SDS gel.

Total recovery of IL-2 protein from the second column was 62%.

Acetonitrile and Endotoxin Removal

The IL-2 pool from the second preparative reversed phase column was phase-separated at -70°C, and the aqueous phase was diluted five-fold with a buffer containing 0.05 M sodium acetate (pH 3.5) and 50 mg/ml mannitol. The diluted IL-2 was then applied to a CM-Fractogel® column (10 cm wide x 34 cm high) which had previously been equilibrated with the

After the IL-2 had been loaded onto the column material, the column was washed with 5 bed volumes dilution buffer. The bound IL-2 was then eluted from the column with a buffer containing 0.05 M sodium acetate (pH 3.5), 50 mg/ml mannitol and 0.2 M NaCl. The column was run at a flow rate of 50 ml/minute and 2,700 ml fractions were collected. The absorbance of the column effluent was monitored at a wave-length of 270 nm. The protein peak recovered was pooled.

The pooled fractions containing IL-2 were concentrated to a final protein concentration of 5-10 mg/ml by ultrafiltration using a YM5 filter with a 5,000 dalton molecular weight cutoff (available from Amicon, Div. W.R.

Grace & Co., Danvers, Massachusetts, U.S.A.) at 2-8 °C. The concentrated protein solution was diafiltered against a buffer containing 50 mM sodium acetate (pH 3.6) and 5 mg/ml mannitol.

Bioassay of the final endotoxin free IL-2 showed that the specific activity of the IL-2 was $1.6 \times 10^7$ activity units/mg protein.

Many modifications and variations of this invention can be made without departing from its spirit and scope, as will be apparent to those skilled in the art. The specific embodiments described herein are offered by way of example only, and the invention is limited only by the terms of the appended claims.

## Claims

1. A process for purifying recombinant human IL-2 comprising:

(a) disrupting cultured bacterial cells containing a vector capable of directing expression of recombinant human IL-2, to produce a bacterial cell lysate containing such IL-2;

(b) isolating an insoluble fraction from the bacterial cell lysate;

(c) extracting the isolated insoluble fraction from the bacterial cell lysate with a solution of from about 4 to 7 M guanidine-HCl to obtain an IL-2 containing extract;

(d) diluting the IL-2 containing extract from about 10- to about 1,000-fold with a guanidine-HCl free buffer;

(e) applying the diluted IL-2 containing extract to a first reversed phase liquid chromatography column whereby the IL-2 is retained by the first column;

(f) eluting the IL-2 from the first column with an increasing acetonitrile gradient and pooling fractions containing IL-2;

(g) applying the pooled IL-2 containing fractions from the first column to a second reversed phase liquid chromatography column whereby the IL-2 is retained by the second column; and (h) eluting the IL-2 from the second column with an increasing acetonitrile gradient and pooling fractions containing IL-2.

2. The process of claim 1 in which the IL-2 containing extract is diluted about 40-fold with the guanidine-HCl free buffer.

3. The process of claim 1 or 2 in which extraction step (c) is preceded by a step comprising extracting the isolated insoluble fraction with a solution containing about 1.75 M guanidine-HCl and discarding the extract.

4. The process of any one of claims 1 to 3 in which the diluted IL-2 containing extract of step (d) is allowed to stand for a period of time ranging from about 4 hours to about 2 days at about 2 to 8 °C prior to application to the first column.

5. The process of claim 4 in which the diluted IL-2 containing extract is allowed to stand for a period of from about 8 to 24 hours.

6. The process of any one of claims 1, to 5 in which the pooled IL-2 from the second column is further purified to remove acetonitrile and endotoxin by chromatography in a carboxymethyl ion exchange column.

FIG 1

FIG 2

FIG 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF CHROMATOGRAPHY, vol. 396, 19th June 1987, pages 209-215, Elsevier Science Publishers B.V., Amsterdam, NL; M.P. WEIR et al.: "Micropreparative purification of recombinant human interleukin-2" * Whole article * | 1-5 | C 12 P 21/02<br>C 07 K 3/08<br>C 07 K 3/12 |
| Y | EP-A-0 254 399 (IMMUNOLOGY VENTURES) * Page 7, lines 35-40 * | 1-6 | |
| Y | BIOCHEMISTRY, vol. 26, no. 11, 2nd June 1987, pages 3129-3134, American Chemical Society, Washington, DC, US; T. TSUJI et al.: "Characterization of disulfide bonds in recombinant proteins: Reduced human interleukin 2 in inclusion bodies and its oxidative refolding" * Whole article * | 1-6 | |
| Y | EP-A-0 147 819 (HOFFMANN LA ROCHE) * Page 19, line 21 - page 21, line 37 * | 1-6 | TECHNICAL FIELDS SEARCHED (Int. Cl.4)<br><br>C 12 P<br>C 07 K |
| D,A | EP-A-0 156 373 (CETUS CORP.) * Page 5, line 17 - page 9, line 32 * | 1-6 | |
| P,X | WO-A-8 808 849 (CETUS CORP.) * Page 19, lines 21-32; claims * | 6 | |

The present search report has been drawn up for all claims

| Place of search | Date of completion of the search | Examiner |
|---|---|---|
| THE HAGUE | 14-07-1989 | HUBER-MACK A. |

EPO FORM 1503 03.82 (P0401)